# EUROPEAN PATENT SPECIFICATION

(11) **EP 1 322 349 B1**
(45) Date of publication and mention of the grant of the patent: **15.06.2005**
(21) Application number: 01973145.4
(22) Date of filing: 19.09.2001
(51) Int. Cl.: A61L 26/00, A61L 28/00

(54) **PROTECTANT FILM FOR SKIN**
SCHUTZFILM FÜR DIE HAUT
FILM DERMO-PROTECTEUR

(30) Priority: 26.09.2000 US 670986
(43) Date of publication of application: 02.07.2003
(73) Proprietor: Coloplast Corporation, North Mankato, MN 56002-8300 (US)
(72) Inventor: GERRISH, Donald, Lee, c/o Coloplast Corporation, North Mankato, MN 56002-8300 (US)
(74) Representative: Nilausen, Kim
(86) International application number: PCT/US2001/029159
(87) International publication number: WO 2002/026278

(56) References cited:
- WO-A-98/42298
- US-A- 4 379 863
- US-A- 4 584 192
- US-A- 5 013 769

## Description

### BACKGROUND OF THE INVENTION

### 1. FIELD OF THE INVENTION.

This invention is directed to a composition that dries to produce a protective film for skin when applied to the skin of a user.

### 2. DESCRIPTION OF THE RELATED ART.

In many circumstances, it is desirable to apply a protectant film to the skin. Such film protectants are particularly desirable in circumstances in which a wound or opening is present for a long period of time and must be protected. Such protectants are particularly useful in connection with incontinence and ostomy appliances, such as are used after a colostomy. Such films should protect the skin against germs, feces, enzymatic drainage or wound drainage, as well as adhesive trauma. Additionally, such films should be flexible on the skin so that they will not crack or flake and should not interfere with adhesion of ostomy appliances, wound care dressing, tape, or attachable breast forms. Moreover, such films should leave a shiny appearance so that one can see that the film has been applied, and should not leave a residue or irritate the skin.

Although a number of films for such uses currently exist, there is a need for films with improved properties that meet the exacting demands of use, particularly in the case of ostomy apertures. There is therefore a need for skin-protecting film-forming polymers with improved properties.

WO 98/42298 discloses wear resistant cosmetic compositions for foundations, blushes or lipsticks and mascaras, comprising a polymer which compositions comprise a styrene-ethylene/propylene mixed copolymer and optionally an alkyl cycloalkylacrylate copolymer may be incorporated. These polymers are insoluble in water and soluble in certain oils and hydrocarbon solvents such as isoparaffin and isododecane.

US Patent No. 4,379,863 discloses a copolymer composition and delivery system for providing a protective barrier film for the skin that protects the skin from attack from acids, caustics and active enzymes found in human waste by providing a waterproof barrier that normally encountered liquids could not penetrate or wash away. The film is formed from a composition comprising a C1-C4 straight chain esters of methacrylic acid dissolved in a volatile organic solvent such as C3-C10 alkanols including cyclohexanol as well as alcohols with an aromatic character, isopropanol being the choice.

US Patent No. 4,584,192 discloses a dermatologically acceptable, film-forming composition containing an antimicrobial agent for use for controlling infection and promoting healing in patients having surgical incisions or other open wounds. The film is fluid resistant and formed from a composition comprising a monomeric acrylic or methacrylic ester of an alcohol containing a single hydroxyl and having from 2 to about 14 carbon atoms, a monomeric methacrylic ester of an alcohol containing a single hydroxyl and having from 1 to about 6 carbon atoms and an N-vinyl lactam and an effective amount of a broad spectrum antimicrobial agent dissolved in a fugitive solvent such as ethanol, isopropanol or acetone.

### SUMMARY OF THE INVENTION

A composition according to the present invention that dries to produce a protective film for skin when applied to the skin of a user comprises:
(1) cycloalkyl methacrylate copolymer;
(2) an evaporative solvent comprising at least one solvent for the cycloalkyl methacrylate copolymer that can evaporate so that the copolymer is deposited on the skin; and
(3) a plasticizer to provide flexibility to the film deposited on the skin.

A preferred cycloalkyl methacrylate copolymer is a bicycloalkyl methacrylate copolymer.

Typically, the solvent system comprises hexamethyldisiloxane and at least one saturated branched-chain hydrocarbon selected from the group consisting of isooctane, isodecane, and isododecane. Preferably, the saturated branched-chain hydrocarbon is isooctane. Alternatively, the saturated branched-chain hydrocarbon can be a mixture of isooctane and isododecane.

Typically, the plasticizer is selected from the group consisting of acetyl tributyl citrate, acetyl triethyl citrate, tributyl citrate, triethyl citrate, acetyl tripropyl citrate, tripropyl citrate, dibutyl sebacate, acetyl dibutyl sebacate, dipropyl sebacate, acetyl dipropyl sebacate, diethyl sebacate, and acetyl diethyl sebacate. Preferably, the plasticizer is acetyl tributyl citrate.

Preferably, the composition comprises from 5 percent by weight to 25 percent by weight of the cycloalkyl methacrylate copolymer, from 73 percent by weight to 94.5 percent by weight of the evaporative solvent, and from 0.5 percent by weight to 2.0 percent by weight of the plasticizer.

Preferably, when the evaporative solvent comprises isooctane and hexamethyldisiloxane, the ratio of isooctane to hexamethyldisiloxane is about 3:5. When the evaporative solvent comprises isooctane, isododecane, and hexamethyldisiloxane, the ratio of isooctane, isododecane, and hexamethyldisiloxane is preferably about 19:11:50.

In a particularly preferred embodiment, the composition comprises:
(1) about 19% by weight cycloalkyl methacrylate copolymer;
(2) about 1% by weight of acetyl tributyl citrate;
(3) about 30% by weight of isooctane; and
(4) about 50% by weight of hexamethyldisiloxane.

In an alternative embodiment, the composition comprises:
(1) about 19% by weight cycloalkyl methacrylate copolymer;
(2) about 1% by weight of acetyl tributyl citrate;
(3) about 19% by weight of isooctane;
(4) about 11% by weight of isododecane; and
(5) about 50% by weight of hexamethyldisiloxane.

It is to be understood that both the foregoing general description and the following detailed description are exemplary and explanatory only and are intended to provide further explanation of the invention as claimed. This specification illustrates several embodiments of the invention and together with the description serve to explain the principles of the invention.

### DETAILED DESCRIPTION OF THE INVENTION

An improved skin-protecting film-forming composition uses the polymer cycloalkyl methacrylate copolymer. This creates a unique protectant film when the polymer is dispersed in a solvent system that evaporates rapidly.

In general, a composition of the present invention is a composition that dries to produce a protectant film for skin when applied to the skin of a user. The composition comprises:
(1) cycloalkyl methacrylate copolymer;
(2) an evaporative solvent comprising at least one solvent for the cycloalkyl methacrylate copolymer that can evaporate so that the copolymer is deposited on the skin; and
(3) a plasticizer to provide flexibility to the film deposited on the skin wherein the solvent system comprises hexamethyldisiloxane and at least one saturated branched-chain hydrocarbon selected from the group consisting of isooctane, isodecane, and isododecane.

A suitable source of a'cycloalkyl methacrylate copolymer that is useful in compositions according to the present invention is Phoenix Chemical, Inc. of Somerville, New Jersey. A particularly suitable cycloalkyl methacrylate copolymer is a bicycloalkyl methacrylate copolymer marketed by Phoenix Chemical as Giovarez AC-5099M.

Preferably, the saturated branched-chain hydrocarbon is a mixture of isooctane and isododecane. Alternatively, the saturated branched-chain hydrocarbon can be isooctane. Other saturated branched-chain hydrocarbons can be used. Preferred saturated branched-chain hydrocarbons include those having low molecular weights and are volatile such that the composition will dry in a short period of time when applied to the skin.

Typically, the plasticizer is selected from the group consisting of acetyl tributyl citrate, acetyl triethyl citrate, tributyl citrate, triethyl citrate, acetyl tripropyl citrate, tripropyl citrate, dibutyl sebacate, acetyl dibutyl sebacate, dipropyl sebacate, acetyl dipropyl sebacate, diethyl sebacate, and acetyl diethyl sebacate. Preferably, the plasticizer is acetyl tributyl citrate. Other plasticizers can be used, including homologues and derivatives of these esters. Preferred plasticizers include those that confer flexibility to the composition when dry and prevent the dried composition from cracking when the skin flexes.

Preferably, the composition comprises from 5% to 25% by weight of the cycloalkyl methacrylate copolymer, from 73% by weight to 94.5% by weight of the evaporative solvent, and from 0.5% by weight to 2.0% by weight of the plasticizer. The amount of the cycloalkyl methacrylate copolymer affects the drying time and the degree of protection of the composition. Compositions having smaller amounts of the cycloalkyl methacrylate copolymer exhibit faster drying times, but provide less protection. Therefore, the optimum amount of the cycloalkyl methacrylate copolymer is based on the desired degree of protection and drying time. The degree of protection and drying times may be determined by methods known in the art.

Where the evaporative solvent comprises isooctane and hexamethyldisiloxane, the ratio of isooctane to hexamethyldisiloxane is preferably about 3:5. When the evaporative solvent comprises isooctane, isododecane, and hexamethyldisiloxane, the ratio of isooctane, isododecane, and hexamethyldisiloxane is preferably about 19:11:50. The ratios of the ingredients or solvents in the solvent system are specific for the particular solvents used. The amounts of certain solvents will depend on the type of solvent, safety standards, skin irritation, and skin defatting. In other words, the amounts of solvents will depend on the solubility, irritability, and volatility of the given solvents. The solubility, irritability, and volatility may be determined by methods known in the art.

In a particularly preferred embodiment, the composition comprises:
(1) about 19% by weight cycloalkyl methacrylate copolymer;
(2) about 1% by weight of acetyl tributyl citrate;
(3) about 30% by weight of isooctane; and
(4) about 50% by weight of hexamethyldisiloxane.

In an alternative embodiment, the composition comprises:
(1) about 19% by weight cycloalkyl methacrylate copolymer;
(2) about 1% by weight of acetyl tributyl citrate;
(3) about 19% by weight of isooctane;
(4) about 11% by weight of isododecane; and
(5) about 50% by weight of hexamethyldisiloxane.

The composition is made by adding the solvents (isooctane and/or isododecane and hexamethyldisiloxane) to the polymer and plasticizer and mixing with vigorous agitation. Methods known in the art may be used to make the composition of the present invention. The degree of agitation required need only to be vigorous enough to ensure that the solvents, polymer and plasticizer are thoroughly mixed.

A composition comprising 30% by weight isooctane, 19% by weight cycloalkyl methacrylate copolymer, 1% by weight acetyl tributyl citrate, and 50% by weight hexamethyldisiloxane was made by mixing 22.5 kg of isooctane and 14.25 kg of cycloalkyl methacrylate copolymer for about 10 to about 15 minutes. Then 0.75 kg of acetyl tributyl citrate was added, and the mixture mixed for about 5 to about 10 minutes. 37.5 kg of hexamethyldisiloxane was then slowly added and mixed for about 15 to about 20 minutes. Mixing was conducted at about 400 to about 1700 rpm.

The polymer is applied to the skin and forms a protective film that can be used, for example, to cover ostomy apertures, wounds, and other openings in the skin. The compositions of the present invention may be applied by various methods with a variety of devices. For example, the compositions may be applied to the skin with a cotton pad or sprayed on the skin with a pump spray bottle. The compositions of the present invention may be washed off the skin with soap and water. However, the composition need not be removed as the composition will wear off with natural skin cell turnover. The composition may be reapplied to the same area.

Preferably, the compositions of the present invention dry rapidly, about 5 to about 60 seconds, preferably, about 5 to about 45 seconds, more preferably, about 5 to about 30 seconds after being applied to the skin. The compositions of the present invention allow the skin underneath the composition to breath. The breathability of the compositions may be determined by methods standard in the art. *See e.g.*, Barlow and Wiechers (1999) Cosmetics & Toiletries 114(12):47-53.

As illustrated in the Examples below, the present invention provides a protectant film that leaves a clear film on the skin. The film is flexible on the skin so that it will not crack or flake. The film does not interfere with adhesion of ostomy appliances, wound care dressing, tape, or attachable breast forms. The film protects skin against urine, feces, enzymatic drainage, would drainage or adhesive trauma. The application of the film leaves a shiny appearance so that one can see that the film has been applied.

The composition of the present invention is non-stinging. The solvent of the composition evaporates rapidly, leaving a dry film. These properties enhance the performance of the composition.

The following examples are intended to illustrate but not to limit the invention.

### Example 1

### Wear-Time Study

The wear-time of the preferred composition (19% by weight cycloalkyl methacrylate copolymer, 1% by weight acetyl tributyl citrate, 30% by weight isooctane, and 50% by weight hexamethyldisiloxane) of the present invention was evaluated by studying the wear-time of the base plate used for ostomy pouches. Twelve (12) healthy subjects who did not have ostomies. wore 2 base plates and closed end pouches, one on each side of the abdomen. On one side, the preferred composition of the present invention was applied and no protective film was applied to the other side. Ostomy appliances were worn for up to 7 days. No significant difference in the wear-time of the base plate was observed between the side having the composition of the present invention as compared to the side without. Therefore, the compositions of the present invention do not affect the adhesion or wear-time of adhesives and adhesive articles such as ostomy appliances.

### Example 2

### Study of Protective and Preventative Characteristics

The efficacy and safety of the preferred composition (about 19% cycloalkyl methacrylate copolymer, about 1% acetyl tributyl citrate, about 30% isooctane, and about 50% hexamethyldisiloxane) of the present invention was determined for use in the treatment of perineal skin breakdown from incontinence. Generally, the reduction in erythema, denudement and pain over a period of 5 days was evaluated. Erythema and denudement was assessed both visually and with photography. Pain was assessed through a subject's verbal and physical communication. Seven (7) subjects having incontinence of urine and feces and having foley catheters were treated with the preferred composition of the present invention. The composition was sprayed around the peritoneal area about every 24 hours. The pain, denudement and erythema decreased in all subjects. Surface area reduction was observed in all of the subjects. Five (5) of the subjects had a decrease in pain on Day 2 and 2 subjects had a decrease in pain on Day 3. Four (4) subjects had no pain by the end of the study period. No adverse events were reported. Therefore, the composition of the present invention may be used to treat or prevent denudement, erythema and pain associated with incontinence. Additionally, the composition of the present invention may be used to treat or prevent exposure of skin to irritants and contaminants.

The clinicians applying the composition noted that they liked the touchless spray system and the fact that the presence of the composition was observable by a shiny appearance unlike creams and other formulations. The subjects requested continued treatment with composition.

## Claims

1. A composition that dries to produce a protective film for skin when applied to the skin of a user comprising:
(a) cycloalkyl methacrylate copolymer;
(b) an evaporative solvent system comprising at least one solvent for the cycloalkyl methacrylate copolymer that can evaporate so that the copolymer is deposited on the skin; and
(c) a plasticizer to provide flexibility to the film deposited on the skin, wherein the solvent system comprises hexamethyldisiloxane and at least one saturated branched-chain hydrocarbon selected from the group consisting of isooctane, isodecane, and isododecane.

2. The composition of claim 1, wherein the at least one saturated branched-chain hydrocarbon is a mixture of isooctane and isododecane.

3. The composition of claim 1, wherein the at least one saturated branched-chain hydrocarbon is isooctane.

4. The composition of claim 1, wherein the plasticizer is selected from group consisting of acetyl tributyl citrate, acetyl triethyl citrate, tributyl citrate, triethyl citrate, acetyl tripropyl citrate, tripropyl citrate, dibutyl sebacate, acetyl dibutyl sebacate, dipropyl sebacate, acetyl dipropyl sebacate, diethyl sebacate, and acetyl diethyl sebacate.

5. The composition of claim 1, wherein the cycloalkyl methacrylate copolymer is a bicycloalkyl methacrylate copolymer.

6. The composition of claim 1, wherein the composition comprises from 5 percent *by weight* to 25 percent *by weight* of the cycloalkyl methacrylate copolymer, from 73 percent *by weight* to about 94.5 percent of the evaporative solvent, and from 0.5 percent *by weight* to 2.0 percent *by weight* of the plasticizer.

7. The composition of claim 1, wherein the evaporative solvent comprises a mixture of isooctane and isododecane, and the plasticizer is acetyl tributyl citrate.

8. The composition of claim 1, wherein the evaporative solvent comprises isooctane and hexamethyldisiloxane, and the plasticizer is acetyl tributyl citrate.

9. The composition of claim 7, wherein the composition comprises from 5 percent *by weight* to 25 percent *by weight* of cycloalkyl
methacrylate copolymer, from about 73 percent *by weight* to 94.5 percent of the evaporative solvent, and from 0.5 percent *by weight* to 2.0 percent *by weight* of the plasticizer.

10. The composition of claim 8, wherein the composition comprises from 5 percent *by weight* to 25 percent *by weight* of the cycloalkyl methacrylate copolymer, from 73 percent *by weight* to 94.5 percent *by weight* of the evaporative solvent, and from 0.5 percent *by weight*
to 2.0 percent *by weight* of the plasticizer.

11. The composition of claim 7, wherein the ratio of isooctane, isododecane, and hexamethyldisiloxane is about 19:11:50.

12. The composition *of claim 1* comprising:
(a) about 19 percent *by weight* of cycloalkyl methacrylate copolymer;
(b) about 1 percent *by weight* of acetyl tributyl citrate;
(c) about 30 percent *by weight* of isooctane; and
(d) about 50 percent *by weight* of hexamethyldisiloxane.

13. The composition *of claim 1* comprising:
(a) about 19 percent *by weight* cycloalkyl methacrylate copolymer;
(b) about 1 percent *by weight* of acetyl tributyl citrate;
(c) about 19 percent *by weight* of isooctane;
(d) about 11 percent *by weight* of isododecane; and
(e) about 50 percent *by weight* of hexamethyldisiloxane.

14. The composition *of claim 1*
wherein the evaporative solvent system is a solvent for the cycloalkyl methacrylate copolymer and can evaporate so that the copolymer is deposited on the skin, the cycloalkyl methacrylate copolymer being soluble in the evaporative solvent system.

## Patentansprüche

1. Zusammensetzung, die beim Aufbringen auf die Haut eines Anwenders zu einem Schutzfilm trocknet und enthält:
(a) ein Cycloalkylmethacrylat-Copolymer,
(b) ein verdampfbares Lösungsmittelsystem, das mindestens ein Lösungsmittel für das Cycloalkylmethacrylat-Copolymer enthält, das verdampfen kann, so dass das Copolymer auf der Haut abgeschieden wird, und
(c) einen Weichmacher, der dem auf der Haut abgeschiedenen Film Flexibilität verleiht,
wobei das Lösungsmittelsystem Hexamethyldisiloxan und mindestens einen gesättigten verzweigten Kohlenwasserstoff enthält, der unter Isooctan, Isodecan und Isododecan ausgewählt ist.

2. Zusammensetzung nach Anspruch 1, bei welcher der mindestens eine gesättigte verzweigte Kohlenwasserstoff ein Gemisch von Isooctan und Isododecan ist.

3. Zusammensetzung nach Anspruch 1, bei welcher der mindestens eine gesättigte verzweigte Kohlenwasserstoff Isooctan ist.

4. Zusammensetzung nach Anspruch 1, bei welcher der Weichmacher ausgewählt ist unter Acetyltributylcitrat, Acetyltriethylcitrat, Tributylcitrat, Triethylcitrat, Acetyltripropylcitrat, Tripropylcitrat, Dibutylsebacat, Acetyldibutylsebacat, Dipropylsebacat, Acetyldipropylsebacat, Diethylsebacat und Acetyldiethylsebacat.

5. Zusammensetzung nach Anspruch 1, bei der das Cycloalkylmethacrylat-Copolymer ein Bicycloalkylmethacrylat-Copolymer ist.

6. Zusammensetzung nach Anspruch 1, die das Cycloalkylmethacrylat-Copolymer in einem Mengenanteil von 5 bis 25 Gew.-%, das verdampfbare Lösungsmittel in einem Mengenanteil von 73 bis etwa 94,5 Gew.-% und den Weichmacher in einem Mengenanteil von 0,5 bis 2 Gew.-% enthält.

7. Zusammensetzung nach Anspruch 1, bei der das verdampfbare Lösungsmittel aus einem Gemisch von Isooctan und Isododecan besteht und der Weichmacher Acetyltributylcitrat ist.

8. Zusammensetzung nach Anspruch 1, bei der das verdampfbare Lösungsmittel aus Isooctan und Hexamethyldisiloxan besteht und der Weichmacher Acetyltributylcitrat ist.

9. Zusammensetzung nach Anspruch 7, die das Cycloalkylmethacrylat-Copolymer in einem Mengenanteil von 5 bis 25 Gew.-%, das verdampfbare Lösungsmittel in einem Mengenanteil von etwa 73 bis 94,5 Gew.-% und den Weichmacher in einem Mengenanteil von 0,5 bis 2,0 Gew.-% enthält.

10. Zusammensetzung nach Anspruch 8, die das Cycloalkylmethacrylat-Copolymer in einem Mengenanteil von 5 bis 25 Gew.-%, das verdampfbare Lösungsmittel in einem Mengenanteil von 73 bis 94,5 Gew.-% und den Weichmacher in einem Mengenanteil von 0,5 bis 2,0 Gew.-% enthält.

11. Zusammensetzung nach Anspruch 7, bei der das Verhältnis von Isooctan, Isododecan und Hexamethyldisiloxan etwa 19:11:50 beträgt.

12. Zusammensetzung nach Anspruch 1, die enthält:
(a) etwa 19 Gew.-% Cycloalkylmethacrylat-Copolymer;
(b) etwa 1 Gew.-% Acetyltributylcitrat;
(c) etwa 30 Gew.-% Isooctan und
(d) etwa 50 Gew.-% Hexamethyldisiloxan.

13. Zusammensetzung nach Anspruch 1, die enthält:
(a) etwa 19 Gew.-% Cycloalkylmethacrylat-Copolymer;
(b) etwa 1 Gew.-% Acetyltributylcitrat;
(c) etwa 19 Gew.-% Isooctan;
(d) etwa 11 Gew.-% Isododecan und
(e) etwa 50 Gew.-% Hexamethyldisiloxan.

14. Zusammensetzung nach Anspruch 1, bei der das verdampfbare Lösungsmittelsystem ein Lösungsmittel für das Cycloalkylmethacrylat-Copolymer ist und verdampfen kann, so dass das Copolymer auf der Haut abgeschieden wird, wobei das Cycloalkylmethacrylat-Copolymer in dem verdampfbaren Lösungsmittelsystem löslich ist.

## Revendications

1. Une composition qui sèche pour produire un film dermo protecteur quand elle est appliquée sur la peau d'un utilisateur, comprenant :
(a) un copolymère de cycloalkylméthacrylate ;
(b) un système de solvant d'évaporation comprenant au moins un solvant pour le copolymère de cycloalkylméthacrylate qui peut s'évaporer de manière à ce que le copolymère se dépose sur la peau ; et
(c) un plastifiant pour apporter de la flexibilité au film déposé sur la peau, dans lequel le système de solvant comprend de l'hexaméthyldisiloxane et au moins un hydrocarbure saturé à chaîne ramifiée choisi dans le groupe constitué de l'isooctane, de l'isodécane et de l'isododécane.

2. La composition de la revendication 1, dans laquelle au moins un hydrocarbure saturé à chaîne ramifiée est un mélange d'isooctane et d'isododécane.

3. La composition de la revendication 1, dans laquelle au moins un hydrocarbure saturé à chaîne ramifiée est l'isooctane.

4. La composition de la revendication 1, dans laquelle le plastifiant est choisi dans le groupe constitué de l'acétylcitrate de tributyle, de l'acétylcitrate de triéthyle, du citrate de tributyle, du citrate de triéthyle, de l'acétylcitrate de tripropyle, du citrate de tripropyle, du sébacate de dibutyle, de l'acétylsébacate de dibutyle, du sébacate de dipropyle, de l'acétylsébacate de dipropyle, du sébacate de diéthyle, et de l'acétylsébacate de diéthyle.

5. La composition de la revendication 1, dans laquelle le copolymère de cycloalkylméthacrylate est un copolymère de bicycloalkylméthacrylate.

6. La composition de la revendication 1, dans laquelle la composition comprend de 5 % en poids à 25 % en poids de copolymère de cycloalkylméthacrylate, de 73 % en poids à 94.5 % en poids de solvant d'évaporation et de 0.5 % en poids à 2,0 % en poids de plastifiant.

7. La composition de la revendication 1, dans laquelle le solvant d'évaporation comprend un mélange d'isooctane et d'isododécane, et le plastifiant est l'acétylcitrate de tributyle.

8. La composition de la revendication 1, dans laquelle le solvant d'évaporation comprend de l'isooctane et de l'hexaméthyldisiloxane, et le plastifiant est l'acétylcitrate de tributyle.

9. La composition de la revendication 7, dans laquelle la composition comprend de 5 % en poids à 25 % en poids de copolymère de cycloalkylméthacrylate, de 73 % en poids à 94.5 % en poids de solvant d'évaporation et de 0.5 % en poids à 2.0 % en poids de plastifiant.

10. La composition de la revendication 8, dans laquelle la composition comprend de 5 % en poids à 25 % en poids de copolymère de cycloalkylméthacrylate, de 73 % en poids à 94.5 % en poids de solvant d'évaporation et de 0.5 % en poids à 2,0 % en poids de plastifiant.

11. , La composition de la revendication 7, dans laquelle le rapport de isooctane/isododécane/hexaméthyldisiloxane est environ 19/11/50.

12. La composition de la revendication 1, comprenant :
(a) environ 19 % en poids de copolymère de cycloalkylméthacrylate ;
(b) environ 1 % en poids d'acétylcitrate de tributyle ;
(c) environ 30 % en poids d'isooctane ; et
(d) environ 50 % en poids d'hexaméthyldisiloxane.

13. La composition de la revendication 1, comprenant :
(a) environ 19 % en poids de copolymère de cycloalkylméthacrylate ;
(b) environ 1 % en poids d'acétylcitrate de tributyle ;
(c) environ 19 % en poids d'isooctane ;
(d) environ 11 % en poids d'isododécane ; et
(e) environ 50 % en poids d'hexaméthyldisiloxane.

14. La composition de la revendication 1, dans laquelle le système de solvant d'évaporation est un solvant pour le copolymère de cycloalkylméthacrylate et peut s'évaporer de manière à ce que le copolymère se dépose sur la peau, le copolymère de cycloalkylméthacrylate étant soluble dans le système de solvant d'évaporation.
